# EUROPEAN PATENT APPLICATION

(11) **EP 1 935 433 A1**
(43) Date of publication of application: **25.06.2008**
(21) Application number: 06025849.8
(22) Date of filing: 13.12.2006
(51) Int. Cl.: A61K 47/48

(54) **A method for preparing covalent lipid-spacer-peptide conjugates**

(71) Applicant: Institute of Nuclear Energy Research, Atomic Energy Council, Taiwan, R.O.C. T'ao yuan County 325 (TW)
(72) Inventor: Lee, Te-Wei, Taipei City 114, Taiwan,R.O.C. (TW); Chiu, Shu-Pei, Gueishan Township, Taoyuan County 333 (TW); Yu, Chiu-Yu, Longtan Township Taoyuan County (TW); Chang, Tsui-Jung, Chiayi City 600 (TW); Chang, Chih-Hsien, Hsinchu City 300 (TW)
(74) Representative: Becker, Eberhard

(57) **Abstract**

The present invention discloses a method for preparing lipid-spacer-reactive functional group-peptide, wherein
the peptide consists of 3 to 16 amino acid residues in which at least one amino acid residue is lysine (Lys),
the reactive functional group is a formula of -X-CO-Y-CO-, wherein X represents an oxygen atom or a nitrogen atom, and Y represents C₁₋₆ alkylene which may be interrupted by one or two oxygen or nitrogen atom(s),
the spacer is a hydrophilic polymer, and
the lipid is phosphatidylethanoaminecarbonyl represented by the formula (I): 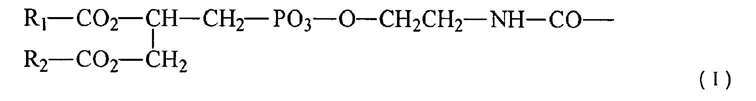 R₁ and R₂ are the same or different and individually represent linear or branch C₇₋₃₀ alkyl or linear or branch C₇₋₃₀ alkenyl; which is characterized in that the reaction is carried out in a liquid phase and comprises the following steps of (a) firstly protecting Lys amino acid residue in the peptide through a protection group; (b) subsequently reacting the peptide with the lipid-spacer-reactive functional group; and (c) finally removing the protection group from Lys amino acid residue in the peptide.

## Description

### Field of the Invention

The present invention relates to a method for synthesizing lipid-spacer-reactive functional group-peptide in a liquid phase. The method can prepare products in high yield and thus can be used in production and synthesis on large scale.

### Background of the Invention

In 1965, liposome was first discovered by Alec Bangham in Babraham Insititue, Cambridge, United Kingdom. Liposome is a lipid-based hollow microsphere encapsulated by phospholipid and cholesterol as membrane materials and having size of about 0.0025µm to 3.5µm and suspending in an aqueous phase. The lipid membrane (microsphere surfaces) presents in the form of lipid bilayers that are mainly constituted at a phosphoric site in the phospholipid molecule. In the phospholipid molecule, because the phosphoric site is hydrophilic and the lipid site is hydrophobic, the outward phosphoric site and the inward lipid site can form a membrane that double sides are hydrophilic and an inner sandwich is hydrophobic. A solution of water soluble material can be encapsulated in the center of the microsphere, and oil soluble material can be sandwiched in the membrane of the microsphere surface. Therefore, liposome can encapsulate water soluble material and oil soluble material as a carrier.

According to the above characteristics of liposome, after 1970s, liposome is gradually considered as a carrier to deliver drugs, especially anticancer drugs. Liposome can encapsulate the anticancer drugs and then target on a region having cancer cells and finally release the anticancer drugs, which cannot easily enter into normal tissues but directly act on the tumor region and thus reduce damage to normal cells. Main advantages of liposome can be described as follows.
1. Drugs encapsulated in liposome can change pharmacokinetics and prolong half life of the drugs in blood. Liposome with size of about 100nm can penetrate holes located at new blood vessel walls of tumors, and a large number of liposome encapsulating the anticancer drugs can be accumulated in the tumors, and hence treatment effect can be increased. This liposome belongs to a passive target.
2. Liposome encapsulating drugs with high toxicity can diminish harmful side effect.
3. Liposome can be applied in various situations since it is very flexible in constituents of lipid, its particle size, its structure, its preparation methods, and its ways used to encapsulate drugs.
4. Since liposome contains phospholipid which is the same as constituents of cell membrane and degradable in an organism, it neither has toxicity nor causes an immunoreaction like proteins and can be used many times.

In order to increase specific interaction between liposome and targeting tissues, a cell-specific ligand can bind to liposome to improve the interaction between liposome and targeting cells, and raise phagocytic ability of cancer cells to liposome, and accomplish release of drugs at a defined position, and diminish non-specific toxicity of anticancer drugs to normal tissues, and increase anticancer effect. Generally, a monoclonal antibody or a ligand can bind to liposome through a covalent bond which is further recognized by a receptor or an antigen on cell surface and then enters into a specific cell. This targeting liposome has better treatment effect than non-targeting liposome.

For example, octreotide is a somatostatin analog having a cyclic structure with eight amino acid residues. Octreotide is an effective inhibitor for growth hormones, glucagons and insulin. Liposome bound with octreotide can form targeting liposome in which one of important components is lipid-polyethyleneglycol-octreotide. Chen *et al.* discloses a method for preparing lipid-polyethyleneglycol-octreotide in USP 6552007B2 and its synthetic process can be described as a chemical reaction shown in the following scheme. 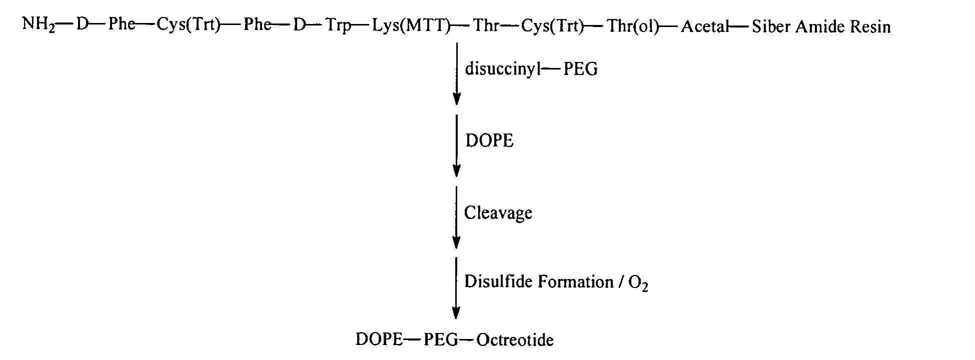 Wu *et al.* also discloses a method for preparing lipid-polyethyleneglycol-octreotide in EP 1319667A2 as a chemical reaction shown in the following scheme. 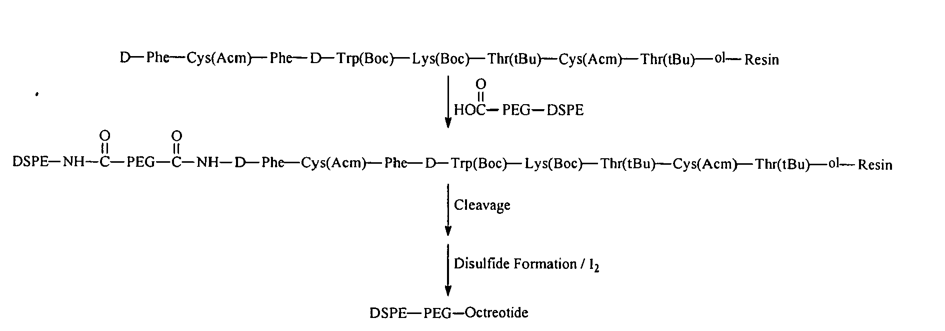 The foregoing methods for preparing lipid-polyethyleneglycol-peptide carry out the synthetic reaction in a solid phase. The synthetic reaction in the solid phase is time-consuming and wastes lots of manufacturing cost because it needs at least six steps to finish which is very complicated. Moreover, many factors can affect yield of synthesizing lipid-polyethyleneglycol-peptide in the solid phase and usually depend on numbers of amino acid residues, ways used to cleave, and conditions used in cyclization and purification. Specifically, the more numbers the amino acid residues have, the lower yield lipid-polyethyleneglycol-peptide can be obtained. Because lipid and polyethyleneglycol are comparatively large molecules, steric hindrance happens at the last step of conducting the cleavage and the cycliztion of the peptide and prolongs reaction time and lowers yield. Furthermore, a solid-phase synthetic instrument is restricted by a maximum synthetic amount of 1 mmole in every batch, and hence it cannot prepare lipid-polyethyleneglycol-peptide in the solid phase on large scale.

To overcome various deficiencies of synthesizing lipid-polyethyleneglycol-peptide in the solid phase, the present invention provides a method for preparing lipid-spacer-reactive functional group-peptide in a liquid phase which carries out the synthetic reaction in an aprotic solvent. The method of the present invention has advantages of simple steps and high yield, and hence it can save lots of cost and can be used in production on large scale.

### Brief Description of the Invention

The present invention discloses a method for preparing lipid-spacer-reactive functional group-peptide, wherein
the peptide consists of 3 to 16 amino acid residues in which at least one amino acid residue is lysine (Lys),
the reactive functional group is a formula of -X-CO-Y-CO-, wherein X represents an oxygen atom or a nitrogen atom, and Y represents C₁₋₆ alkylene which may be interrupted by one or two oxygen or nitrogen atom(s),
the spacer is a hydrophilic polymer, and
the lipid is a phosphatidylethanoaminecarbonyl represented by the formula (I): 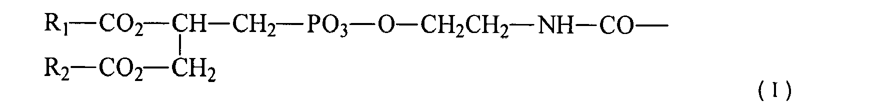 R₁ and R₂ are the same or different and individually represent linear or branch C₇₋₃₀ alkyl or linear or branch C₇₋₃₀ alkenyl;
which is characterized in that the reaction is carried out in a liquid phase and comprises the following steps of (a) firstly protecting Lys amino acid residue in the peptide through a protection group; (b) subsequently reacting the peptide with the lipid-spacer-reactive functional group; and (c) finally removing the protection group from Lys amino acid residue in the peptide.

### Detailed Description of the Invention

According to the method for preparing lipid-spacer-reactive functional group-peptide of the present invention, the peptide consists of 3 to 16 amino acid residues in which at least one amino acid residue is Lys. The amino acid residues are selected from at least one group consisting of alanine (Ala), cysteine (Cys), glycine (Gly), lysine (Lys), phenylalanine (Phe), threonine (Thr), tryptophan (Trp), tyrosine (Tyr), and valine (Val). The amino acid residues can arrange in the presence of a straight line or a cyclic form. Preferably, the peptide is a somatostatin analog consisting of 6 to 14 amino acid residues in which at least one amino acid residue is Lys. Embodiment of the peptide is for instance, but not limited to, c[N-methyl-Ala-Tyr-D-Trp-Lys-Val-Phe] (seglitide), D-Phe-c[Cys-Phe-D-Trp-Lys-Thr-Cys]-Thr(ol) (octreotide), Tyr³-octreotide, D-Phe¹-octreotide, DβNal-c[Cys-Tyr-D-Trp-Lys-Val-Cys]-Thr(ol) (lanreotide), D-Phe-c[Cys-Tyr-D-Trp-Lys-Val-Cys]-Trp (vapreotide), D-Phe-c[Cys-Phe-Gly-Lys-Thr-Cys]-Thr(ol), etc.

According to the method for preparing lipid-spacer-reactive functional group-peptide of the present invention, the reactive functional group represented by a formula of -X-CO-Y-CO- binds to the spacer, wherein X represents an oxygen atom or a nitrogen atom, and Y represents C₁₋₆ alkylene which may be interrupted by one or two oxygen or nitrogen atom(s). A carboxyl site in the reactive functional group is used to generate a bond of -CONH- with the peptide, and the other site X in the reactive functional group can bond to the spacer, and thus the spacer and the peptide are connected. Embodiment of the reactive functional group is derived from, for instance, but not limited to, succinic acid, succinic anhydride, N-hydroxylsuccinimide, etc.

According to the method for preparing lipid-spacer-reactive functional group-peptide of the present invention, the function of the spacer is to connect the hydrophilic peptide and the hydrophobic lipid, and hence a hydrophilic polymer with a long chain is suitable to be used. Embodiment of the spacer is derived from, for instance, but not limited to, polyvinylpyrrolidine, polymethacrylate, polyethyloxazoline, polyvinylmethylether, polypropyleneglycol, polyethyleneglycol, etc. The spacer is preferably derived from polyethyleneglycol (PEG) having a formula of -(CH₂CH₂O)ₘ-, wherein m is 34 to 46. The spacer is more preferably derived from PEG600, PEG2000 or PEG3000.

According to the method for preparing lipid-spacer-reactive functional group-peptide of the present invention, R₁ and R₂ showing in phosphatidylethanoaminecarbonyl represented by the formula (I) are the same or different and individually represent C₇₋₃₀ alkyl or C₇₋₃₀ alkenyl, preferably C₁₂₋₁₄ alkyl or C₁₂₋₁₄ alkenyl, both of which are in the presence of linear form or branch form. Embodiment of R₁ and R₂ are for instance, but not limited to, dodecyl, myristyl, palmitoyl, stearyl, oleyl, erucyl, etc. Preferably, R₁ and R₂ are stearyl or oleoyl.

According to the method for preparing lipid-spacer-reactive functional group-peptide of the present invention, the kind of the protection group described in steps (a) and (c) and ways used to bind the protection group to and remove the protection group from the amino acid residue are conventional art and can be determined based on the amino acid residue to be protected and its position in the peptide chain by persons skilled in the art. In the method of the present invention, embodiment of the protection group used to protect Lys amino acid residue in the peptide is for instance, but not limited to, t-butyloxycarbonyl (Boc), 2-chlorobenzyloxycarbonyl (2-CIZ), 9-fluorenylmethyloxycarbonyl (Fmoc), allyloxycarbonyl (Aloc), 1-(4,4-dimethyl-2,6-dioxocyclohexylidene)ethyl (Dde), 1-(1'-adamantyl)-1-methylethoxycarbonyl (Adpoc), etc.

According to the method for preparing lipid-spacer-reactive functional group-peptide of the present invention, steps (a), (b) and (c) are all conducted under circumstances of the liquid phase. Steps (a) and (b) are conducted by dissolving the peptide and lipid-spacer-reactive functional group in an aprotic solvent. Embodiment of the aprotic solvent is for instance, but not limited to, N,N-dimethylformamide (DMF), N,N-dimethylacetamide (DMAc), tetrahydrofurane (THF), dimethylsulfoxide (DMSO), hexamethylphosphoramide (HMPA), acetonitril (ACN), etc. Preferably, the aprotic solvent is N,N-dimethylformamide and tetrahydrofurane.

According to the method for preparing lipid-spacer-reactive functional group-peptide of the present invention, the amount of the lipid-spacer-reactive functional group and the peptide can be determined based on numbers of the carboxyl group in the reactive functional group and numbers of the amino group in the peptide. Preferably, the reaction can be conducted by maintaining the reactive functional group / the peptide in the ratio of 4/1 to 1/4, more preferably in the ratio of 1/2.

According to the method for preparing lipid-spacer-reactive functional group-peptide of the present invention, all steps are conducted at a temperature of from 15 to 50°C, preferably 20 to 35°C. Steps (a) and (b) are individually conducted the reaction for 12 to 36 hours, preferably 20 to 28 hours.

According to the method for preparing lipid-spacer-reactive functional group-peptide of the present invention, when the amino acid residues in the peptide arrange in the presence of a straight line, the peptide can optionally further conduct cyclization reaction. Cyclization reaction for peptide is well known by persons skilled in the art and can be conducted during or after any step of steps (a), (b) and (c).

The products produced by the method for preparing lipid-spacer-reactive functional group-peptide of the present invention can be used as a main component of targeting liposome.

### Embodiment

To understand the present invention completely, the preparation method will be illustrated in detail in the following examples. However, those well known to persons skilled in the art will not be described in detail to avoid restricting the scope of the present invention inappropriately. The scope of the present invention will be defined in the claims.

Abbreviations used in the specification of the present invention are listed as follows.
- PEG: polyethyleneglyol
- DSPE: distearoyl phosphatidylethanolamine
- DOPE: dioleoyl phosphatidylethanolamine
- Boc: t-butyloxycarbonyl
- SA: succinic anhydride
- DSPC: distearoyl phosphatidylcholine

### Embodiment 1 : Synthesis of D-Phe-c[Cys-Phe-D-Trp-Lys(Boc)-Thr-Cys]-Thr(ol)

Into a round bottom flask, 100mg of octreotide was put and dissolved by adding 5mL of N,N-dimethylformamide. After the octreotide completely dissolved, 20µL of (Boc)₂O was added to the flask. The mixture reacted at room temperature for 24 hours and then the solvent was extracted by using a vacuum system to obtain a crude product, D-Phe-c[Cys-Phe-D-Trp-Lys(Boc)-Thr-Cys]-Thr(ol). Finally, the crude product was purified by a high performance liquid chromatography through a column of Hibar 250-10 Lichrosorb RP-18 (7µm) produced by Merck & Co., Inc., with 0.1% of trifluoroacetic acid/H₂O as an eluent and 40 minutes (80% ~ 10%) as analytic time. The resultant retention time was 29.4 minutes. The solution from the major peak was collected and then subjected to freeze dry to obtain solid white powder (72mg, yield: 70%) which was analyzed mass spectrometry as [M+H]⁺=1119Da.

### Embodiment 2 : Synthesis of DSPE-PEG-SA

15g of DSPE and 3.9g carbonyl dimidazole were mixed and dissolved in 70mL of toluene. 2g of triethylamine was further added and then the mixture reacted at a temperature of 100°C for one hour. On the other hand, 40g of PEG having average molecular weight of 2000 dissolved in 15mL of toluene which was then gradually dropped into the above mixture and kept carrying out the reaction. The solvent was removed after the reaction finished. The resultant solid product dissolved in 500mL of acetone and the insoluble solid was removed by filtration. The filtrate was extracted to dry, and the resultant solid product was exchanged to Na⁺ form by using cation exchange resin, and DSPE-PEG-OH was thus obtained. Subsequently, 2.1g of succinic anhydride dissolved in 100mL of toluene solution containing 1.7g of pyridine and kept reacting with DSPE-PEG-OH. 500mL of ethylether (about 5 times total volume of the reaction solution) was added, and then the obtained solid product was DSPE-PEG-SA. Afterward, the product was separated through a column of Silica Gel 60 balanced by chloroform and having 63 to 200µm of particle size and 1.5x30cm length, with 4/1 of chloroform/methanol as an eluent. The product was confirmed its location and purity by a TLC method and then collected by removing the solvent through a depression dry method. The product was analyzed mass spectrometry as [M+H]⁺=2892Da, and the analytic result of nuclear magnetic resonance (¹H NMR) was as follows.
¹H NMR (300MHz, CDCl₃)
δ0.78 ~ 1.40 (66H, dialkyl H)
δ2.33 (4H, brt, CO-CH₂)
δ3.64 (160H, brs, PEG-H)
δ3.85 ~ 4.50 (9H, m, glycerol-H and O-CH₂-CH₂-N)

### Embodiment 3 : Synthesis of DSPE-PEG-octreotide

100mg of DSPE-PEG-SA and 34.6mg of D-Phe-c[Cys-Phe-D-Trp-Lys(Boc)-Thr-Cys]-Thr(ol) were mixed and dissolved in 6mL of N,N-dimethylformamide. After the solid completely dissolved, 4.1mg of N-hydroxybenzotriazole and 6.4mg of dicyclohexylcarbodiimide were added to the above solution and reacted together for 24 hours, and the solvent was then removed by extraction. To remove the protection group Boc in D-Phe-c[Cys-Phe-D-Trp-Lys(Boc)-Thr-Cys]-Thr(ol), 5mL of 95% trifluoroacetic acid was added to the mixture and reacted for 30 minutes, and the solvent was then removed. An excess of chloroform was added, and the solution kept standing to carry out precipitation and was then filtered through a filter paper No.42. The filtrate was concentrated, and an excess of chloroform was further added, and then the foregoing steps were repeated several times. A compound of DSPE-PEG-octreotide, a light brown solid (122mg, yield: 91%), was obtained by concentrating the filtrate through a decompressing concentrator. The product was analyzed by a high performance liquid chromatography with the same analytic conditions that Example 1 used except for using 0.1% of trifluoroacetic acid/CH₃CN as the eluent. The resultant retention time was 14.5 minutes. The product was analyzed mass spectrometry as [M+H]⁺=3893Da, and the analytic result of nuclear magnetic resonance (¹H NMR) was as follows.
¹H NMR (300MHz, CDCl₃)
δ0.84 ~ 1.40 (66H, dialkyl H)
δ3.64 (160H, brs, PEG-H)
δ3.85 ~ 4.50 (9H, m, glycerol-H and O-CH₂-CH₂-N)
δ8.04 (4H, benzyl)

### Embodiment 4 : Targeting Liposome (Octreotide-Liposome-Doxorubicin) Synthesized by Encapsulating Doxorubicin through a Thin-Film Hydration Method

DSPC (70□mole) / cholesterol / DSPE-PEG2000 / DSPE-PEG2000-octreotide (3 : 2 : 0.094 : 0.206 in molar ratio), DSPC (70□mole) / cholesterol / DSPE-PEG2000-octreotide (3 : 2 : 0.206 in molar ratio), and DSPC (70□mole) / cholesterol / DSPE-PEG2000-octreotide (3 : 2 : 0.3 in molar ratio) were added separately into 250mL of round bottom flask and dissolved in 8mL of chloroform individually. After the mixture dissolved completely, the organic solvent was extracted by a spin-decompressing concentrator under a vacuum at a temperature of 60□. After the chloroform was removed completely, a lipid membrane was formed on the wall of the flask. 5mL of 250mM ammonium sulfate solution (pH5.0, 530mOs) was then added to the round bottom flask contained the lipid membrane. The flask was shaken and vibrated in a water bath at a temperature of 60□ until all of the lipid membrane on the wall of the flask was dispersed into the ammonium sulfate solution, and multilayer liposome (multilamellar vesicle, MLV) was thus obtained. The multilayer liposome suspension was repeatedly frozen under liquid nitrogen and thawed in a water bath at a temperature of 60□ for six times, and then filtered and extruded by a high pressure filter extruder (Lipex Biomembrane Inc., Vancouver, Canada) to obtain monolayer liposome.

Doxorubicin was subsequently encapsulated with every one micromole of phospholipid to 140g of doxorubicin. A doxorubicin stock with the concentration of 10mg/mL formulated in advance was added to the liposome suspension and reacted at a temperature of 60□ under 100rpm for 30 minutes. After the reaction finished, the suspension was immediately placed onto ice bucket. In order to remove un-encapsulated doxorubicin, the liposome suspension encapsulating the doxorubicin then passed through a gel filtration column of Sephadex G50 with 0.9% of sodium chloride as an eluent. The liposome suspension passing through the column was collected and centrifugated by an ultracentrifuge under 150000xg for 90 minutes. Most supernatant was removed and a small amount of supernatant was left, and then the precipitated liposome was re-suspended evenly. The liposome suspension was filtered by a 0.22µm filter to obtain the final product, Octreotide-Liposome-Doxorubicin. The doxorubicin encapsulated in the liposome then subjected to the concentration measurement and the particle size analysis.
1. The average particle size of the liposome was measured 75 to 95nm in a normal distribution by a N4 Plus particle size analyzer.
2. The concentration of doxorubicin encapsulated in the liposome was measured 2mg/mL by a fluorescence spectrophotometer (JASCO, FP6200) with an excitation wavelength of 475nm and an emission wavelength of 580nm.

### Embodiment 5 : Cell Uptake Activity Assay for Targeting Liposome

This experiment was designed to analyze cellular uptake of targeting-liposome-drugs, Octreotide-Liposome-Doxorubicin, in pancreatic AR42J tumor cell line. An appropriate amount of daughter cells was sub-cultured to carry out the experiment. AR42J cells were firstly cultured in a 6-well culture plate with the concentration of 5×10⁵ cells/well and adhered overnight. After the cells adhered, one control (1 mL/well HBSS) and three experimental (Free Doxorubicin, Liposome-Doxorubicin (INER), and Octreotide-Liposome-Doxorubicin) drug sets were added with the concentration of 1mL/well for 2 and 4 hours. After the reaction finished, the cells was washed with phosphate buffer saline (PBS) to stop the drug reaction. Subsequently, 5% of sodium dodecyl sulfate (SDS) was used to lyse the cells for 10 minutes in order to release the drugs that had been uptaken. After the released drugs that had been uptaken were sufficiently mixed, 1mL of the released drugs were placed into a disposal cuvette and analyzed a spectrum with an excitation wavelength of 475nm and an emission wavelength of 580nm, based on a characteristic that the doxorubicin could generate auto-fluorescence. Meanwhile, the lysed cells were normalized by protein quantification. Finally, the concentration of the drug uptake was compared with the normalized numbers of the cells to obtain the numbers of the cellular drug uptake. Various experimental groups were compared to each other.

Table 1 shows cellular uptake of various doxorubicin formulations in AR42J. Because the Control set was treated with HBSS, there was no doxorubicin uptake, demonstrating that the doxorubicin uptake depends on the presence of doxorubicin. Free Doxorubicin had the highest uptake in the cells wherein the cellular uptake after 2 hours was 96.43x10⁹ molecules/cell and the cellular uptake after 4 hours was 110.83×10⁹ molecules/cell. After 2 and 4 hours, the cellular uptake of Liposome-Doxorubicin (INER) with a liposome characteristic was 1.89×10⁹ molecules/cell and 2.46×10⁹ molecules/cell, respectively. Tumor targeting drugs, Octreotide-Liposome-Doxorubicin, had different cellular uptake after 2 and 4 hours according to various formulations: Octreotide-Liposome-Doxorubicin were individually 1.97×10⁹ molecules/cell and 3.14×10⁹ molecules/cell; Octreotide-Liposome-Doxorubicin 4% were individually 2.26×10⁹ molecules/cell and 3.43×10⁹ molecules/cell; and Octreotide-Liposome-Doxorubicin_6% were individually 2.98×10⁹ molecules/cell and 5.35×10⁹ molecules/cell.

**Table 1: Comparison of cellular uptake of various doxorubicin formulations in AR42J**

| Doxorubicin formulations | Cellular doxorubicin uptake (×10⁹ molecules/cell) (n=3) | |
|---|---|---|
| | 2hr | 4hr |
| Control | 0 | 0 |
| Free Doxorubicin | 96.43 ± 5.75 | 110.83 ± 0.62 |
| Liposome-Doxorubicin (INER) | 1.89 ± 0.07 | 2.46 ± 0.03 |
| Octreotide-Liposome-Doxorubicin | 1.97 ± 0.14 | 3.14 ± 0.17 |
| Octreotide-Liposome-Doxorubicin_4% | 2.26 ± 0.29 | 3.43 ± 0.14 |
| Octreotide-Liposome-Doxorubicin_6% | 2.98 ± 0.32 | 5.38 ± 0.38 |

## Claims

1. A method for preparing lipid-spacer-reactive functional group-peptide, wherein said peptide consists of 3 to 16 amino acid residues in which at least one amino acid residue is lysine (Lys),
said reactive functional group is a formula of -X-CO-Y-CO-, wherein X represents an oxygen atom or a nitrogen atom, and Y represents C₁₋₆ alkylene which may be interrupted by one or two oxygen or nitrogen atom(s),
said spacer is a hydrophilic polymer, and
said lipid is phosphatidylethanoaminecarbonyl represented by the formula (I): 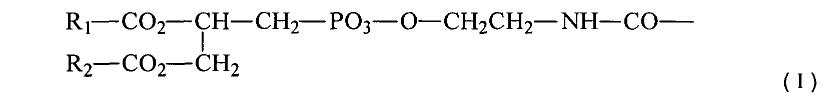 R₁ and R₂ are the same or different and individually represent linear or branch C₇₋₃₀ alkyl or linear or branch C₇₋₃₀ alkenyl;
which is **characterized in that** the reaction is carried out in a liquid phase and comprises the following steps of (a) firstly protecting Lys amino acid residue in said peptide through a protection group; (b) subsequently reacting said peptide with said lipid-spacer-reactive functional group; and (c) finally removing said protection group from Lys amino acid residue in said peptide.

2. The method according to Claim 1, wherein said amino acid residues in said peptide are selected from at least one group consisting of alanine (Ala), cysteine (Cys), glycine (Gly), lysine (Lys), phenylalanine (Phe), threonine (Thr), tryptophan (Trp), tyrosine (Tyr), and valine (Val).

3. The method according to Claim 1, wherein said amino acid residues in said peptide arrange in the presence of a straight line or a cyclic form.

4. The method according to Claim 1, wherein said peptide is a peptide consisting of 6 to 14 amino acid residues in which at least one amino acid residue is Lys.

5. The method according to Claim 4, wherein said peptide is selected from at least one group consisting of seglitide, octreotide, Tyr³-octreotide, D-Phe¹-octreotide, lanreotide, and vapreotide.

6. The method according to Claim 1, wherein said reactive functional group is derived from at least one group consisting of succinic acid, succinic anhydride, and N-hydroxylsuccinimide.

7. The method according to Claim 1, wherein said spacer is derived from at least one group consisting of polyvinylpyrrolidine, polymethacrylate, polyethyloxazoline, polyvinylmethylether, polypropyleneglycol, and polyethyleneglycol.

8. The method according to Claim 7, wherein said spacer is derived from polyethyleneglycol having a formula of -(CH₂CH₂O)ₘ-, wherein m is 34 to 46.

9. The method according to Claim 8, wherein said spacer is derived from PEG600, PEG2000 or PEG3000.

10. The method according to Claim 1, wherein R₁ and R₂ showing in phosphatidylethanoaminecarbonyl represented by the formula (I) individually represent linear or branch C₁₂₋₂₄ alkyl or linear or branch C₁₂₋₂₄ alkenyl.

11. The method according to Claim 9, wherein R₁ and R₂ showing in phosphatidylethanoaminecarbonyl represented by the formula (I) are selected from at least one group consisting of dodecyl, myristyl, palmitoyl, stearyl, oleyl, and erucyl.

12. The method according to Claim 1, wherein in said step (a), said protection group used to protect said Lys amino acid residue in said peptide is selected from at least one group consisting of t-butyloxycarbonyl, 2-chlorobenzyloxycarbonyl, 9-fluorenylmethyloxycarbonyl, allyloxycarbonyl, 1-(4,4-dimethyl-2,6-dioxocyclohexylidene)ethyl, and 1-(1'-Adamantyl)-1-methylethoxycarbonyl.

13. The method according to Claim 1, wherein said steps (a) and (b) are conducted in an aprotic solvent.

14. The method according to Claim 13, wherein said aprotic solvent is selected from at least one group consisting of N,N-dimethylformamide, N,N-dimethylacetamide, tetrahydrofurane, dimethylsulfoxide, hexamethylphosphoramide, and acetonitril.

15. The method according to Claim 1, wherein the amount of said lipid-spacer-reactive functional group and said peptide can maintain said reactive functional group / said peptide in the ratio of 4/1 to 1/4

16. The method according to Claim 1, wherein said steps (a), (b) and (c) are conducted at a temperature of from 15 to 50□.

17. The method according to Claim 1, wherein said steps (a) and (b) are individually conducted for 12 to 36 hours

18. The method according to Claim 1, wherein said peptide further conducts cyclization reaction during or after any step of steps (a), (b) and (c).

19. A targeting liposome, which is obtained from lipid-spacer-reactive functional group-peptide produced by the method according to Claim 1 as a main component.

## Amended claims

### Amended claims in accordance with Rule 137(2) EPC.

**1.** A method for preparing lipid-spacer-reactive functional group-peptide, wherein
said peptide consists of 3 to 16 amino acid residues in which at least one amino acid residue is lysine (Lys),
said reactive functional group is a formula of -X-CO-Y-CO-, wherein X represents an oxygen atom or a nitrogen atom, and Y represents C₁₋₆ alkylene which may be interrupted by one or two oxygen or nitrogen atom(s),
said spacer is derived from the group consisting of polyvinylpyrrolidine, polymethacrylate, polyethyloxazoline, polyvinylmethylether, polypropyleneglycol, and polyethyleneglycol, and
said lipid is phosphatidylethanoaminecarbonyl represented by the formula (I): 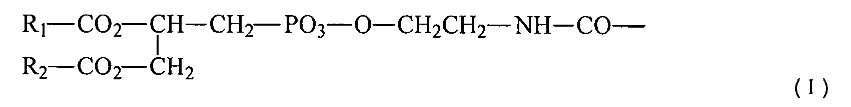 R₁ and R₂ are the same or different and individually represent linear or branch C₇₋₃₀ alkyl or linear or branch C₇₋₃₀ alkenyl;
which is **characterized in that** the reaction is carried out in a liquid phase and comprises the following steps of (a) firstly protecting Lys amino acid residue in said peptide through a protection group; (b) subsequently reacting said peptide with said lipid-spacer-reactive functional group; and (c) finally removing said protection group from Lys amino acid residue in said peptide.

**2.** The method according to Claim 1, wherein said amino acid residues in said peptide are selected from the group consisting of alanine (Ala), cysteine (Cys), glycine (Gly), lysine (Lys), phenylalanine (Phe), threonine (Thr), tryptophan (Trp), tyrosine (Tyr), and valine (Val).

**3.** The method according to Claim 1, wherein said amino acid residues in said peptide arrange in the presence of a straight line or a cyclic form.

**4.** The method according to Claim 1, wherein said peptide is a peptide consisting of 6 to 14 amino acid residues in which at least one amino acid residue is Lys.

**5.** The method according to Claim 4, wherein said peptide is selected from the group consisting of seglitide, octreotide, Tyr³-octreotide, D-Phe¹-octreotide, lanreotide, and vapreotide.

**6.** The method according to Claim 1, wherein said reactive functional group is derived from the group consisting of succinic acid, succinic anhydride, and N-hydroxylsuccinimide.

**7.** The method according to Claim 1, wherein said spacer is derived from polyethyleneglycol having a formula of -(CH₂CH₂O)ₘ-, wherein m is 34 to 46.

**8.** The method according to Claim 7, wherein said spacer is derived from PEG600, PEG2000 or PEG3000.

**9.** The method according to Claim 1, wherein R₁ and R₂ showing in phosphatidylethanoaminecarbonyl represented by the formula (I) individually represent linear or branch C₁₂₋₂₄ alkyl or linear or branch C₁₂₋₂₄ alkenyl.

**10.** The method according to Claim 10, wherein R₁ and R₂ showing in phosphatidylethanoaminecarbonyl represented by the formula (I) are selected from the group consisting of dodecyl, myristyl, palmitoyl, stearyl, oleyl, and erucyl.

**11.** The method according to Claim 1, wherein in said step (a), said protection group used to protect said Lys amino acid residue in said peptide is selected from the group consisting of t-butyloxycarbonyl, 2-chlorobenzyloxycarbonyl, 9-fluorenylmethyloxycarbonyl, allyloxycarbonyl, 1-(4,4-dimethyl-2,6-dioxocyclohexylidene)ethyl, and 1-(1'-Adamantyl)-1-methylethoxycarbonyl.

**12.** The method according to Claim 1, wherein said steps (a) and (b) are conducted in an aprotic solvent selected from the group consisting of N,N-dimethylformamide, N,N-dimethylacetamide, tetrahydrofurane, dimethylsulfoxide, hexamethylphosphoramide, and acetonitril.

**13.** The method according to Claim 1, wherein the ratio of said lipid-spacer-reactive functional group and said peptide is maintained between 4/1 and 1/4

**14.** The method according to Claim 1, wherein said steps (a), (b) and (c) are conducted at a temperature of from 15 to 50°C.

**15.** The method according to Claim 1, wherein said steps (a) and (b) are individually conducted for 12 to 36 hours

**16.** The method according to Claim 1, wherein said peptide further conducts cyclization reaction during or after any step of steps (a), (b) and (c).

**17.** A targeting liposome, which is obtained from lipid-spacer-reactive functional group-peptide produced by the method according to anyone of the preceding Claims 1 to 16 as a main component.
